# FASCICULE DE BREVET EUROPEEN

(11) **EP 3 890 697 B1**
(45) Date de publication et mention de la délivrance du brevet: **09.04.2025**
(21) Numéro de dépôt: 19868209.8
(22) Date de dépôt: 04.12.2019
(51) Int. Cl.: A61K 8/73, A61K 8/34, A61K 8/02, A61K 8/00, A61Q 1/00, A61Q 19/00, A61Q 11/00

(54) **COMPOSITIONS COSMETIQUES COMPRENANT UNE CYCLODEXTRINE AYANT UNE DISTRIBUTION DE TAILLE DE PARTICULE**
KOSMETISCHE ZUSAMMENSETZUNGEN, BESTEHEND AUS EINEM ZYKLODEXTRIN MIT EINER PARTIKELGRÖSSENVERTEILUNG
COSMETIC COMPOSITIONS COMPRISING A CYCLODEXTRINE HAVING A PARTICLE SIZE DISTRIBUTION

(30) Priorité: 05.12.2018 FR 1872343; 05.11.2019 FR 1912367
(43) Date de publication de la demande: 13.10.2021
(73) Titulaire: ROQUETTE FRERES, 62136 Lestrem (FR)
(72) Inventeur: MENTINK, Léon, 59000 LILLE (FR); PIOT, Sophie, 75017 PARIS (FR)
(74) Mandataire: Plasseraud IP
(86) Numéro de dépôt international: PCT/FR2019/052932
(87) Numéro de publication internationale: WO 2020/115438

(56) Documents cités:
- WO-A1-94/22501
- US-A1- 2004 234 479
- MAURER KARINE: "Etude rhéologique et texturale de dispersions alimentaires", 24 April 2018 (2018-04-24), XP093077431, Retrieved from the Internet <URL:https://hal.univ-lorraine.fr/tel-01776362/document> [retrieved on 20230830]
- GILBERT LAURA: "Caractérisation physico-chimique et sensorielle d'ingrédients cosmétiques: une approche méthodologique", 10 December 2012 (2012-12-10), XP093077434, Retrieved from the Internet <URL:https://theses.hal.science/tel-00956601> [retrieved on 20230830]

## Description

### Domaine technique

L'invention porte sur des compositions cosmétiques ou dermatologiques comprenant au moins une cyclodextrine dispersée sous forme de particules solides, lesdites particules présentant une taille moyenne en volume d(4,3) mesurée par granulométrie laser, inférieure ou égale à 12 µm et une distribution de taille en volume, mesurée par granulométrie par diffraction laser, dont les diamètres caractéristiques d(10), d(50) et d(90) sont tels que le diamètre d(10) est inférieur ou égal à 2,5 µm, le diamètre d(50) est inférieur ou égal à 10 µm, et le diamètre d(90) est inférieur ou égal à 25,0 µm. La cyclodextrine est de ce fait présente en une quantité supérieure à sa solubilité dans le milieu physiologiquement acceptable de ces compositions. De telles compositions présentent un profil sensoriel amélioré, notamment en ce qui concerne le toucher. L'invention porte aussi sur l'utilisation de ladite cyclodextrine afin d'améliorer le profil sensoriel de compositions cosmétiques, et comme poudre sensorielle en cosmétique.

Les compostions cosmétiques selon l'invention sont susceptibles d'être utilisées dans tous les domaines de la cosmétique, notamment dans le soin et la protection de la peau, l'hygiène corporelle, les soins capillaires, les soins bucco-dentaires, le maquillage et les parfums.

### Technique antérieure

Un produit cosmétique doit répondre aux besoins des consommateurs, qui exigent à la fois efficacité, sécurité, et propriétés sensorielles. Les perceptions sensorielles constituent l'attrait principal du consommateur pour le produit utilisé, bien avant que les effets bénéfiques soient observables. Le défi que doit relever un produit cosmétique est donc de délivrer un effet cosmétique bénéfique tout en fournissant, avant ou pendant l'application, des sensations les plus agréables possibles. Afin d'obtenir un profil sensoriel intéressant et attractif, les formulations des produits cosmétiques se sont au cours du temps complexifiées en intégrant toujours plus d'ingrédients fonctionnels ou sensoriels d'origine synthétique, généralement issus de la pétrochimique.

Toutefois, les produits cosmétiques doivent aujourd'hui répondre à une nouvelle attente des consommateurs : l'origine naturelle, et de façon encore plus exigeante, la naturalité des compositions. En effet, les consommateurs recherchent désormais des produits cosmétiques composés pour l'essentiel d'ingrédients naturels ou d'origine naturelle, présentant le minimum de modifications chimiques ou de greffons de synthèse ou d'origine pétrochimique.

La suppression d'ingrédients fonctionnels ou sensoriels issus de la pétrochimie, ou leur substitution par des ingrédients d'origine naturelle, constitue un axe important de développement de nouveaux produits cosmétique. Cependant, l'introduction de ces nouveaux ingrédients naturels, ou d'origine naturelle, peut s'accompagner d'une dégradation des propriétés sensorielles du produit cosmétique de manière plus ou moins marquée, au niveau de leur aspect, de leur prise, de leur application ou de leur propriétés une fois appliqués sur la peau ou les phanères. Ainsi, les compositions répondant aux critères de naturalité peuvent s'avérer difficiles à étaler, pelucher ou procurer, à l'application, une sensation de crissant, ou une sensation de cassant, ou encore une sensation de glissant insuffisante. Ces perceptions sensorielles insuffisantes ou dégradées sont préjudiciables à la qualité ou à l'image d'un produit cosmétique.

Dans le domaine des crèmes pour le soin de la peau par exemple, notamment celles se présentant sous la forme d'émulsions huile-dans-eau, les ingrédients fonctionnels couramment utilisés sont des émulsifiants, qui sont des tensioactifs issus de la pétrochimie. La substitution de ces tensioactifs par des composés naturels est encore un sujet de recherche active. Un type de système émulsifiant a été développé par la demanderesse, et fait l'objet d'une demande de brevet FR1762841 non publiée à ce jour. Ce système émulsifiant est un mélange d'une bêta-cyclodextrine et d'un agent émulsifiant naturel eau dans huile, tel que par exemple le polyglycéryl-3 diisostéarate, qui permet d'ajuster au mieux les propriétés physico-chimiques et sensorielles des compositions et d'obtenir des émulsions huile dans eau de viscosité adaptée et stable. L'utilisation de cyclodextrines, ayant une taille de particules moyenne en volume inférieure à 12µm, pour le contrôle des mauvaises odeurs est divulgué dans la demande WO94/22501.

En réalisant des émulsions, par exemple des crèmes, il a été constaté avec ce système émulsifiant naturel développé par la demanderesse, des perceptions sensorielles très intéressantes et acceptables, mais tout à fait perfectibles. Comparativement à des formulations comprenant des émulsifiants pétrochimiques, les crèmes comprenant ce système émulsifiant naturel peuvent, sur certaines peaux, être moins faciles à étaler, moins pénétrantes, susceptibles de former des peluches, et peuvent présenter un toucher moins doux et moins poudré, et parfois plus gras.

Les ingrédients sensoriels sont des ingrédients ajoutés à la composition cosmétique pour en ajuster le profil sensoriel au travers d'effets physiques. Parmi les plus utilisés, on trouve le talc, généralement employé pour fournir un toucher doux, poudré et velouté, tout en permettant une absorption des lipides, notamment ceux d'origine naturelle tels que le sébum généré par la peau, ou ceux introduits volontairement dans une composition cosmétique. Or, les récentes études pointent une possible toxicité du talc à l'état de poudre sèche, ce qui pousse l'industrie cosmétique à rechercher des alternatives naturelles à cet ingrédient. Dans un souci d'efficacité, il est notamment recherché un substitut qui reproduise la perception sensorielle au toucher que le talc et sa capacité d'absorption des lipides.

### Résumé de l'invention

La présente invention a pour objet, selon un premier aspect, une composition cosmétique ou dermatologique comprenant au moins une cyclodextrine présente, au moins partiellement sous forme de particules solides dispersées dans un milieu physiologiquement acceptable, lesdites particules présentant une taille moyenne en volume d(4,3) mesurée par granulométrie laser, inférieure ou égale à 12 µm, et préférentiellement inférieure ou égale à 8 µm et une distribution de taille en volume, mesurée par granulométrie par diffraction laser, dont les diamètres caractéristiques d(10), d(50) et d(90) sont tels que :
a. le diamètre d(10) est inférieur ou égal à 2,5 µm,
b. le diamètre d(50) est inférieur ou égal à 10,0 µm, et
c. le diamètre d(90) est inférieur ou égal à 25,0 µm.
L'invention propose aussi une solution pour améliorer les propriétés sensorielles des produits cosmétiques au moyen d'une telle cyclodextrine.

La demanderesse a en effet découvert, de manière surprenante, que l'utilisation d'une cyclodextrine sous forme d'une poudre, composée de particules de taille sélectionnée, et de préférence sélectionnées selon leur distribution de taille particulière, permettait l'obtention de compositions cosmétiques présentant des propriétés sensorielles très avantageuses et améliorées, en particulier en terme de facilité d'étalement, et de toucher doux, poudré, et non gras.

Dans les compositions cosmétiques, les cyclodextrines sont usuellement utilisées comme agent stabilisateur de molécule active, ou comme agent améliorant la biodisponibilité de molécule active, ou encore comme agent favorisant la dispersibilité ou la solubilité dans l'eau de molécules actives. Les cyclodextrines sont ainsi connues pour améliorer l'efficacité des actifs cosmétiques, voire permettre d'en observer les effets. Cependant, les cyclodextrines ne sont pas connues pour améliorer les propriétés sensorielles des compositions cosmétiques ou dermatologiques.

Au sens de la présente invention, on entend par composition ou produit cosmétique ou dermatologique, tout mélange de matières premières destiné à être mis en contact avec les matières kératiniques (telles que la peau, les cheveux, les poils, les ongles, les lèvres et les organes génitaux externes, ou avec les dents et les muqueuses buccales en vue, exclusivement ou principalement, de les nettoyer, de les parfumer, d'en modifier l'aspect, de les protéger, de les maintenir en bon état ou de corriger les odeurs corporelles. Cette définition inclut également les compositions dermo-cosmétiques. Elle exclut toutefois les articles d'hygiène tels que les serviettes hygiéniques ou les couches.

Un second objet de l'invention porte sur l'utilisation, dans une composition cosmétique ou dermatologique, d'au moins une cyclodextrine, au moins partiellement présente sous forme de particules solides dispersées dans un milieu physiologiquement acceptable, caractérisée en ce que les particules de cyclodextrine présentent une taille moyenne en volume d(4,3), mesurée par granulométrie laser, inférieure ou égale à 12 µm, et préférentiellement inférieure ou égale à 8 µm, et une distribution de taille en volume, mesurée par granulométrie par diffraction laser, dont les diamètres caractéristiques d(10), d(50) et d(90) sont tels que :
a. le diamètre d(10) est inférieur ou égal à 2,5 µm,
b. le diamètre d(50) est inférieur ou égal à 10,0 µm, et
c. le diamètre d(90) est inférieur ou égal à 25,0 µm
pour améliorer les propriétés sensorielles de ladite composition, préférentiellement pour conférer une facilité d'étalement, et/ou un toucher doux, poudré, non gras, et/ou prévenir la formation de peluches.

Un troisième objet de l'invention porte sur un procédé de préparation desdites compositions cosmétiques contenant une cyclodextrine selon l'invention..

### Description de l'invention

### La cyclodextrine

La composition selon l'invention met en œuvre au moins une cyclodextrine présente, au moins partiellement, sous forme de particules solides.

Dans la présente Demande, le terme « cyclodextrine » désigne et inclut l'une quelconque des cyclodextrines connues par ailleurs, telles que les cyclodextrines natives et non substituées contenant de 6 à 12 unités de glucose liées par des liaisons covalentes entre les carbones 1 et 4, et notamment les alpha-, beta- et gamma- cyclodextrines contenant respectivement 6, 7 et 8 unités de glucose.

Ce terme couvre également les « dérivés de cyclodextrine » à savoir des molécules dont une partie au moins des groupements hydroxyles OH a été transformée en groupements OR, où R désigne de manière générale un groupement alkyl. De ce point de vue, les dérivés de cyclodextrine incluent notamment les cyclodextrines méthylées, éthylées, mais aussi celles substituées avec un groupement hydroxyalkyl telles que les cyclodextrines hydroxypropylées et hydroxyéthylées.

Toutefois, en raison de leur caractère totalement naturel, les cyclodextrines préférées selon la présente invention sont les alpha-, beta- et gamma- cyclodextrines natives, c'est-à-dire non modifiées.

Selon un mode préféré de réalisation, la cyclodextrine mise en œuvre dans la composition selon l'invention est une bêta-cyclodextrine, de préférence « native .

La cyclodextrine pourra notamment se présenter sous forme d'une poudre cristalline, pseudo-cristalline ou amorphe.

La cyclodextrine peut être sous une forme dite « libre », c'est-à-dire une forme où sa cavité est vide, ou sous la forme connue de complexe d'inclusion. Préférentiellement, la cyclodextrine est sous une forme libre.

Dans les compositions cosmétiques selon l'invention, la cyclodextrine est présente au moins partiellement sous la forme de particules solides. Par « au moins partiellement », la demanderesse entend de préférence qu'au moins 5 % en poids de cyclodextrine, par rapport au poids total de cyclodextrine dans la composition, est présente sous forme de particules solides. Préférentiellement, au moins 10 % en poids de cyclodextrine, par rapport au poids total de cyclodextrine dans la composition, est présente sous forme de particules solides, plus préférentiellement au moins 15 % en poids, encore plus préférentiellement au moins 25 % en poids, toujours plus préférentiellement au moins 50% en poids, encore plus préférentiellement au moins 75 % en poids, et tout préférentiellement au moins 95 % en poids.

Selon un mode de réalisation, la teneur en cyclodextrine sous forme de particules solides dans la composition cosmétique ou dermatologique, va de 0,1 % à 100 % en poids, par rapport au poids total de la composition cosmétique. Préférentiellement, cette teneur va de 0,1 à 50 % en poids, de préférence de 0,1 % à 25 % en poids, plu préférentiellement de 0,1 % à 10 % en poids, et encore plus préférentiellement de 0,1 % à 5 % en poids.

Outre sa présence sous forme solide, la cyclodextrine peut être présente dans la composition selon l'invention sous forme dissoute, en raison de l'équilibre liquide-solide, à l'état libre ou à l'état de complexe d'inclusion, mais la cyclodextrine dissoute ne confère pas aux compositions les propriétés sensorielles améliorées obtenues avec les particules solides de cyclodextrine précédemment décrites.

De manière connue, ou facilement accessible par des mesures simples, la solubilité d'une cyclodextrine dans un milieu physiologiquement acceptable, qu'il comprenne une phase aqueuse et/ou huileuse, est la valeur de la concentration en cyclodextrine dissoute pour laquelle le milieu physiologiquement acceptable est saturé en cyclodextrine. Quand la concentration en cyclodextrine dépasse cette solubilité, autrement dit quand le milieu physiologiquement acceptable est « à saturation » en cyclodextrine, la cyclodextrine ne se dissout plus dans le milieu, et des particules solides de cyclodextrine se dispersent en conservant leur état de solide divisé.

Ainsi, pour garantir une teneur minimale en cyclodextrine sous forme de particules solides, il est possible de considérer la solubilité de ladite cyclodextrine, de préférence à 20°C, dans la composition cosmétique. Ainsi, selon un mode préféré de réalisation, la teneur en cyclodextrine dans la composition selon l'invention est supérieure à la solubilité de ladite cyclodextrine dans le milieu physiologiquement acceptable de la composition cosmétique. En particulier, la teneur en cyclodextrine dans la composition peut être supérieure de 5% à sa solubilité dans le milieu physiologiquement acceptable de ladite composition, de préférence supérieure de 10% à sa solubilité dans ledit milieu, plus préférentiellement supérieure de 15 % à sa solubilité dans ledit milieu, tout préférentiellement supérieure de 25% à sa solubilité dans ledit milieu, et plus préférentiellement supérieure de 50% à sa solubilité dans ledit milieu.

En particulier, la teneur totale en cyclodextrine présente dans la composition cosmétique ou dermatologique selon l'invention représente de 1 à 20 % en poids, par rapport au poids total du milieu physiologiquement acceptable de ladite composition, de préférence de 5 à 15% en poids. Dans le cas particulier où la cyclodextrine est une bêta-cyclodextrine, et où le milieu physiologiquement acceptable est constitué d'eau, la cyclodextrine est de préférence native, et la teneur totale de cyclodextrine représente plus que 1,8 % en poids, par rapport au poids total d'eau présente dans la composition, valeur correspondant approximativement à la solubilité de la bêta-cyclodextrine dans l'eau à 20°C.

Lorsque la composition cosmétique est une composition pulvérulente, dite « sèche » en ce sens qu'elle se présente sous forme d'une poudre ou d'un mélange de poudres, la solubilité de la cyclodextrine peut être considérée comme égale à zéro. En effet, les compositions cosmétiques pulvérulentes sont généralement constituées d'un mélange d'ingrédients solides sous forme de poudres, pouvant contenir une humidité résiduelle allant de 0,01 % à 20 %, par exemple au plus 16 % pour la bêta-cyclodextrine, et optionnellement d'un liant gras non aqueux. Cette humidité est l'eau liée aux poudres, et n'est pas disponible pour une quelconque solubilisation, et donc, notamment, ne permet pas de solubiliser la cyclodextrine. Ainsi, dans le cas de ces compositions cosmétiques sèches, la cyclodextrine est toujours présente en une quantité supérieure à sa solubilité dans lesdites compositions.

Dans le cadre de la présente invention, la cyclodextrine est présente dans la composition cosmétique ou dermatologique, au moins partiellement, sous forme de particules solides, de taille moyenne en volume d(4,3), mesurée par granulométrie laser, inférieure ou égale à 12 µm, et préférentiellement inférieure ou égale à 8 µm.

Selon un mode préférentiel de réalisation, la taille moyenne en volume des particules solides de cyclodextrine est comprise entre 3 µm et 12 µm, et préférentiellement entre 4 µm et 8 µm.
Les particules solides de cyclodextrine sont caractérisées par une distribution de taille en volume, mesurée par granulométrie par diffraction laser, dont les diamètres caractéristiques d(10), d(50) et d(90) sont tels que :
- le diamètre d(10) est inférieur ou égal à 2,5 µm,
- le diamètre d(50) est inférieur ou égal à 10,0 µm, et
- le diamètre d(90) est inférieur ou égal à 25,0 µm.

Les diamètres caractéristiques d(10), d(50) et d(90), tels que définis dans la norme ISO 13320 :2009 sous les notations x10, x50 et x90, sont les diamètres de particules correspondant respectivement à 10 %, 50 % et 90 %, de la distribution de taille cumulative en volume.

De manière encore plus préférentielle, les particules solides de cyclodextrine sont caractérisées par une distribution de taille en volume, mesurée par granulométrie par diffraction laser, ayant un coefficient de variation inférieur ou égal à 73 %. Tel que défini dans la norme ISO 13320 :2009, le coefficient de variation de la distribution de taille de particule est l'écart type de la distribution de taille de particule divisé par la taille moyenne en volume d(4,3), aussi appelé diamètre moyen en volume.

Les particules solides de cyclodextrines peuvent être de toutes formes géométriques, régulières ou irrégulières, et peuvent être des cristaux de cyclodextrine bien individualisés ou des agglomérats de cristaux de cyclodextrine liés entre eux par des ponts cristallins.

La taille moyenne en volume, généralement notée noté d4,3 ou d(4,3), est déterminée à partir des distributions de taille en volume mesurées par granulométrie par diffraction laser, par exemple à l'aide d'un granulomètre laser de la gamme MasterSizer^{®}, par exemple « Mastersizer 2000^{™} », « Mastersizer 3000^{™} », « Mastersizer 3000E^{™} », de la société Malvern Instruments^{®}, ou d'un granulomètre laser « Particula LA960 » de la société Horiba^{©}. Ces méthodes de mesures par diffraction laser peuvent être mises en œuvre en voie humide ou en voie sèche. Lorsque la voie humide est utilisée, il est recommandé d'utiliser le 2-propanol comme fluide de mesure. Selon ce mode préférentiel de réalisation, la cyclodextrine se présente donc sous forme de particules solides dont la distribution de taille en volume mesurée par granulométrie par diffraction laser, présente les caractéristiques suivantes :
a. Une taille moyen en volume d(4,3) inférieure ou égale à 12 µm, et préférentiellement inférieure ou égale à 8 µm,
b. Un coefficient de variation inférieur ou égal à 73 %.

### Domaines de la cosmétique concernés par l'invention

Les compostions cosmétiques selon l'invention sont susceptibles d'être utilisées dans tous les domaines du cosmétique, que sont le soin et la protection de la peau, l'hygiène corporelle, les soins capillaires, les soins bucco-dentaires, les parfums, eaux de toilette et eaux de Cologne, les soins dépilatoires, et le maquillage et démaquillage pour paupières, cils, joues, lèvres, ongles, ou autres parties du corps.

Préférentiellement, les compositions cosmétiques selon l'invention sont utilisées dans le soin et la protection de la peau, les soins capillaires, les soins bucco-dentaires, le maquillage et démaquillage, et les soins dépilatoires.

Dans le domaine du soin de la peau ou des lèvres, les compositions cosmétiques selon l'invention peuvent être les crèmes, émulsions, lotions, gels et huiles pour la peau, les produits anti-âge ou antirides, les masques de beauté, les produits solaires, les produits auto-bronzants, les produits de blanchiment de la peau ou encore les baumes à lèvres.

Dans le domaine de l'hygiène corporelle et du soin du corps, les compositions cosmétiques selon l'invention peuvent être les savons de toilette, les savons liquides, les savons déodorants, les gels douche, les shampoings, les poudres de bain, les préparations pour bain et douches sous forme de sels, de mousses, d'huiles, les produits d'hygiène dentaire comme les dentifrices et les bains de bouche, et les produits pour le rasage, les produits de démaquillage.

Dans le domaine capillaires, les compositions cosmétiques peuvent être les produits pour l'ondulation ou le défrisage, ou les produits de coiffage, les produits de mise en plis, les produits pour le nettoyage du cheveu tels que les lotions ou les poudres ou les shampoings, les produits d'entretien pour la chevelure tels que les lotions ou les crèmes ou les huiles, les produits de coiffage sous forme de lotions ou de laques ou de brillantines, ou les produits de coloration capillaire.

Dans le domaine du maquillage, les compositions cosmétiques selon l'invention peuvent être les fonds de teints sous forme liquide, pâte ou poudre, ainsi que les produits de maquillage destinés à être appliqués sur les cils, les ongles, les joues ou les lèvres tels que les rouges à lèvres.

### Poudre sensorielle

Dans la composition cosmétique selon l'invention, la cyclodextrine remplit la fonction de poudre sensorielle. Par « poudre sensorielle », la demanderesse entend désigner un ingrédient cosmétique se présentant sous forme solide, préférentiellement sous forme d'une poudre, ayant des caractéristiques de forme, d'état de surface, de structure ou propriétés mécaniques, notamment la souplesse et la dureté, et de granulométrie, lui conférant des effets sensoriels bénéfiques. Dans le cadre de la présente invention, ces effets sensoriels bénéfiques peuvent être une diminution de la sensation de gras, un toucher plus sec et plus doux, et un fini non collant.

### Galénique

Les compositions cosmétiques selon l'invention peuvent se présenter sous la forme d'un gel tel qu'un gel aqueux ou hydroalcoolique, d'une dispersion liquide aqueuse et/ou huileuse, telle qu'une émulsion, une suspension, une lotion, un sérum, un aérosol ou une mousse, ou encore sous la forme d'une pâte ou d'une poudre, ou sous la forme d'un solide.

Selon un mode de réalisation de l'invention, la composition cosmétique est une émulsion eau-dans-huile ou une émulsion huile-dans-eau. Alors que les émulsions eau-dans-huile apportent un toucher gras, glissant et non doux du fait de leur aptitude à former un film lipidique à la surface de la peau, les émulsions huile-dans-eau fournissent généralement un toucher plus doux et moins gras que celles-ci. Les émulsions peuvent se présenter sous différentes formes d'usage selon leur formulation, de fluide à épais : sérum, lait, mousse, crème fluide ou épaisse, ou gel. De telles émulsions ont de multiples champs d'application : laits visage et corps, produits solaires (laits et émulsions pulvérisables), maquillage (mascaras, fonds de teint, ombres à paupières, crème), crème de soins pour le visage, cold-creams, crèmes pour le corps et les mains, capillaire (après-shampooings, shampooings, crèmes et masques crèmes).

Préférentiellement, l'émulsion selon l'invention est une émulsion huile-dans-eau dans laquelle la teneur totale en cyclodextrine est comprise entre 1,8 % et 10 % en poids, par rapport au poids total d'eau. Préférentiellement dans ce mode de réalisation, ladite cyclodextrine est une bêta-cyclodextrine. Selon un mode préféré de réalisation, l'émulsion peut être une émulsion stabilisée par un système émulsifiant constitué de bêta-cyclodextrine selon l'invention et d'un émulsifiant d'origine naturelle tel que défini ci-après.

Selon un autre mode de réalisation, la composition cosmétique selon l'invention est une dispersion liquide, aqueuse ou grasse, comme par exemple une lotion, qui contient une phase liquide, aqueuse ou grasse, et une phase pulvérulente, ladite phase pulvérulente comprenant au moins une cyclodextrine présente au moins partiellement sous forme de particules solides selon l'invention.

Selon un autre mode de réalisation, la composition cosmétique selon l'invention est une pâte à usage cosmétique, se présentant comme un milieu semi-solide constitué d'une phase continue hautement visqueuse dans laquelle sont dispersées des particules solides de cyclodextrine. Elle peut par exemple prendre la forme d'un dentifrice, ou d'un baume à lèvres.

Selon un autre mode de réalisation, la composition cosmétique selon l'invention est un solide, tel qu'un rouge à lèvre, un stick déodorant, ou du savon.

Selon un autre mode de réalisation, la composition cosmétique selon l'invention est une poudre à usage cosmétique, qui comprend une phase pulvérulente comportant des pigments et des charges, et une phase grasse comprenant des corps gras, agissant comme liants pour conférer au produit fini des propriétés définies de densité, d'émollience et de douceur lors de l'application. Une telle composition poudreuse prend la forme de fard à paupières, de fard à joues, de produit anticerne, de poudre du visage et du corps, et de produit de maquillage du visage et du corps.

Dans ce cas de composition cosmétique sous forme de poudre, la composition peut être obtenue par un procédé de type « slurry » c'est-à-dire par dispersion d'une phase pulvérulente dans une phase solvant hydroalcoolique, puis évaporation de la phase solvant.

Ainsi, selon un mode préféré de l'invention, la composition cosmétique ou dermatologique se présente sous la forme d'un produit de soin de la peau, de soin des cheveux, de soin bucco-dentaire, un maquillage, ou un soin dépilatoire.

### Composants de la composition cosmétique

### Milieu physiologiquement acceptable

La composition selon l'invention comprend également un milieu physiologiquement acceptable, et de préférence cosmétiquement acceptable, c'est-à-dire qui ne présente pas d'effets secondaires délétères et en particulier qui ne produit pas de rougeurs, d'échauffements, de tiraillements ou de picotements inacceptables pour un utilisateur de produits cosmétiques.

Le milieu physiologiquement acceptable comprend par exemple une phase grasse et/ou une phase aqueuse.

### Phase grasse

La phase grasse peut renfermer au moins une huile.

Au sens de la présente invention, on entend par « huile » un composé liquide à température ambiante (25°C), et qui, lorsqu'il est introduit à raison d'au moins 1% en poids dans l'eau à 25°C, n'est pas du tout soluble dans l'eau, ou soluble à hauteur de moins de 10% en poids, par rapport au poids d'huile introduit dans l'eau.

La phase grasse liquide comprend avantageusement une ou plusieurs huiles non volatiles qui procurent un effet émollient sur la peau. On peut citer les esters gras tels que l'isononoate de cetearyl, l'isononoate d'isotridecyl, l'isostearate d'isostearyl, l'isostearate d'isopropyl, le myristate d'isopropyl, le palmitate d'isopropyl, stéarate de butyle, le laurate d'hexyle, l'isononate d'isononyle, le palmitate de 2-éthyl-hexyle, le laurate de 2-hexyldécyle, le palmitate de 2-octyl décyle, le myristate ou la lactate de 2-octyldodécyle, le succinate de 2-diéthyl hexyle, le malate de diisostéaryle, la tracétine , la tricprine , les triglycerides d'acide caprylique/caprique, le mélange de caprate et caprylate de coco, les benzoates d'alcools en C12 à C15, les esters de glycol tels que le butylène glycol cocoate, le triisostéarate de glycérine, l'acetate de tocopherol, les acides gras supérieurs tels que l'acide myristique, l'acide palmitique, l'acide stéarique, l'acide béhénique, l'acide oléique, l'acide linoléique, l'acide linolénique ou l'acide isostéarique, les alcools gras supérieurs tel que l'alcool oléique, les huiles végétales comme l'huile d'avocat, l'huile de camélias, l'huile de noisette, l'huile de tsubaki, l'huile de noix de cajou, l'huile d'argan, l'huile de soja, l'huile de pépins de raisin, l'huile de sésame, l'huile de mals, l'huile de germes de blé, l'huile de colza, l'huile de tournesol, l'huile de coton, l'huile de jojoba, l'huile d'arachide, l'huile d'olive et leurs mélanges, les beurres végétaux comme le beurre de karité, le beurre de camellia.

Selon un mode préféré de réalisation, la composition selon l'invention comprend une huile non volatile choisie parmi le mélange de caprate et caprylate de coco, les esters de glycol tels que le butylène glycol cocoate et leur mélange.

Ces huiles peuvent être des huiles de type hydrocarbures ou siliconnées telles que l'huile de paraffine, de squalane, la vaseline, les diméthyls siloxanes et leurs mélanges.

La phase grasse liquide peut aussi comprendre éventuellement des huiles volatiles. Par huile volatile, on entend une huile susceptible de s'évaporer de la peau, en moins d'une heure à température ambiante et pression atmosphérique. Les huiles volatiles peuvent être par exemple choisies parmi les huiles de silicones ou triglycérides d'acides gras courts pour réduire le toucher gras.

De préférence la composition selon l'invention ne contient que des huiles d'origine renouvelable et notamment des huiles d'origine végétale, de préférence raffinées.

La quantité d'huile contenue dans la composition selon l'invention représente de préférence de 10 à 80%, du poids total de cette composition.

La phase grasse de la composition selon l'invention peut en outre avantageusement renfermer au moins un agent structurant de phase grasse tel qu'un gélifiant lipophile.

La phase grasse peut également renfermer une ou plusieurs cires et/ou un ou plusieurs composés pâteux.

On entend par "composé pâteux" des corps gras lipophiles qui, comme les cires, sont capables de subir un changement d'état liquide/solide réversible et ont à l'état solide une organisation cristalline anisotrope, mais qui se différencient des cires par le fait qu'ils renferment, à une température de 23°C, une fraction liquide et une fraction solide.

Par « cire », on entend un corps gras à changement d'état liquide / solide réversible, ayant une température de fusion supérieure à 30°C et généralement inférieure à 90°C, qui est liquide dans les conditions de préparation de la composition et présente à l'état solide une organisation cristalline anisotrope. Les cires utilisées selon l'invention peuvent être constituées de cires polaires ou apolaires ou d'un mélange des deux. Par « apolaire », on entend une cire ne renfermant que des atomes de carbone, d'hydrogène et/ou de phosphore et en particulier un hydrocarbure.

Les cires polaires peuvent notamment être choisies parmi les cires animales, les cires végétales et les cires synthétiques ou de silicone renfermant des groupements polaires tels que les esters. On peut ainsi mentionner les cires de Carnauba, de Candelilla, d'abeille (Cera alba), d'insecte de Chine (Ericerus pela); du Japon, de Sumac, de Montan, les triesters d'acides en C8-C20 et de glycérine tels que le tribéhénate de glycérine, le stéarate de glycol acétylé commercialisé notamment par la société VEVY sous la dénomination commerciale CETACENE, et leurs mélanges. Ces cires peuvent notamment être utilisées sous forme prédispersée dans une huile, comme c'est le cas du mélange de cire de candelilla et d'huile de graine de jojoba commercialisé par la société INA TRADING sous la dénomination commerciale GREEN GREASE.

La phase grasse de la composition selon l'invention peut également comprendre au moins un polymère filmogène, susceptible d'apporter de la tenue et/ou des propriétés de non-transfert et/ou de la brillance au maquillage conféré par la composition.

### Phase aqueuse

La composition selon l'invention peut, en outre, comprendre une phase aqueuse comprenant de l'eau et optionnellement, un ou plusieurs solvants organiques miscible à l'eau, et/ou un ou plusieurs agent de rhéologie.

La composition selon l'invention peut notamment comprendre une teneur en eau allant de 10 à 50% en poids, par rapport au poids total de la composition.

L'agent de rhéologie peut notamment être un agent épaississant de la phase aqueuse, un agent gélifiant ou un agent suspensif, tels que par exemple les gommes issues de plantes comme la gomme arabique, la gomme de konjac , la gomme de guar ou leurs dérivés ; les gommes extraits d'algues comme les alginates ou les carraghénanes ; les gommes issues d'une fermentation microbienne comme les xanthanes, les mannanes , les scléroglucanes ou leurs dérivés ; la cellulose et ses dérivés comme la carboxyméthylcellulose ou l'hydroxyéthylcellulose ; l'amidon et ses dérivés comme en particulier les amidons modifiées, notamment acétylés, carboxyméthylés, octénylsuccinates ou hydroxypropylés ; les polymères synthétiques comme les polyacide-acryliques ou les carbomères.

Selon un mode préféré de réalisation, l'agent de rhéologie est la gomme de xanthane.

De préférence la composition selon l'invention comprend un agent de rhéologie choisi parmi les polysaccharides naturels issus de plantes ou de fermentation, éventuellement modifiés.

### Tensioactifs

La composition selon l'invention peut également comprendre un ou plusieurs tensioactifs, choisis de préférence parmi les émulsionnants eau-dans-huile (E/H) ou huile-dans-eau (H/E), de préférence d'origine naturelle.

L'émulsionnant huile-dans-eau peut notamment être choisi parmi les esters de sorbitane éventuellement polyéthoxylés, les esters d'acides gras et de glycérol, les esters ou polyesters d'acides gras et de sucrose, les esters d'acides gras et de polyéthylèneglycol, les polysiloxanes modifiés polyéthers, les éthers d'alcools gras et de polyéthylèneglycol, les alkylpolyglycosides et la lécithine hydrogénée, sans que cette liste ne soit limitative.

L'émulsionnant eau-dans-huile (E/H) peut être choisi parmi les esters gras non éthoxylés de polyols, et notamment parmi les esters gras non éthoxylés de glycérol, de polyglycérols, de sorbitol, de sorbitan, d'anydrodrohexitols comme en particulier l'isosorbide, de mannitol, de xylitol, d'érythritol, de maltitol, de saccharose, de glucose, de polydextrose, de sirops de glucose hydrogéné, de dextrines et d'amidons hydrolysés.

L'émulsionnant E/H peut être choisi parmi les esters gras non éthoxylés de polyols obtenus à partir d'acide gras ou par trans-estérification à partir d'huile ou de mélanges d'huiles. Les acides gras utilisés comprennent de 8 à 22 atomes de carbone, de préférence de 10 à 18 atomes de carbone, et en particulier de 12 à 18 atomes de carbone. Ces acides peuvent être linéaires ou ramifiés, saturés ou insaturés, posséder une ou des fonctions hydroxyles latérales. Les huiles peuvent être saturées ou insaturées, de liquide à solide à température ambiante, et posséder éventuellement des fonctions hydroxyle, de préférence d'indice d'iode compris entre 1 et 145, et en particulier de 5 à 105.

L'émulsionnant E/H d'origine naturelle peut être choisi également parmi les produits naturellement biodégradables en milieu naturel hydraté, avec notamment une balance Hydrophile-Lipophile (HLB) comprise entre 1,5 et 6, de préférence entre 2 et 5 et mieux encore entre 3 et 5.

L'émulsionnant E/H d'origine naturelle peut être en particulier choisi parmi les esters gras de glycérol, et notamment parmi les oléates, stéarates, isostéarates de glycérol comme par exemple les produits suivants : glyceryl laurate HLB 5,2, glyceryl oleate HLB 4 comme IMWITOR 948, glyceryl isostearate Schercemol GMIS HLB 3.5 de Lubrizol Schercemol et glyceryl monostearate HLB 3.5 de Sympatens-GMS.

Il peut être également choisi parmi les esters gras de sorbitan ou de sorbitol, notamment parmi les laurates, palmitate, oléates, stéarate, isostéarates de sorbitan comme par exemple les produits sorbitan trioleate Kosteran-O/3 HLB 1.8 ; sorbitan oleate MONTANE 80 VG ou SPAN 80-LQ-(RB) ou Kosteran-O/1 HLB 4.3 ; sorbitan isostearate Kosteran-I/1 HLB 4.3, sorbitan stearate Kosteran-S/1 HLB 4.7 ; sorbitan monopalmitate HLB 6,6 ; sorbitan laurate Kosteran-L/1 HLB 8.6, .

Il peut être aussi choisi parmi les esters gras de saccharose comme par exemple le distéarate sucrose SP60-C de Sisterna, le polystéarate sucrose SP10-C de Sisterna, le saccharose cocoate HLB 6.

L'émulsionnant E/H d'origine naturelle peut être en particulier choisi parmi les esters de polyglycérols et de préférence parmi les esters issu de la réaction de polyglycérols comprenant de 2 à 12 unités de glycérol, de préférence de 3 à 6 unités glycérol avec au moins une huile végétale partiellement hydrogénée ou non hydrogénée d'indice d'iode compris entre 1 et 1 5, et en particulier de 5 à 10. Il peut s'agir en particulier des esters oléiques , stéariques , isostéariques et ricinoléiques de polyglycérols et en particulier des produits suivants : polyglyceryl-4 isostearate HLB 3 (comme HYDRIOL^{®} PGI de HYDRIOR) , polyglyceryl-10 pentaoleate HLB 3.5 (comme DECAGLYN 5-OV), polyglyceryl-6 polyricinoleate (comme HEXAGLYN PR-15), polyglyceryl-2 sesquiisostearate HLB ~ 4 (comme Hostcerin DGI de Clariant et Dermofeel^{®} GO soft de Evonik Dr. Straetmans), polyglycéryl-3 ricinoleate HLB 3,5 , polyglyceryl-3 polyricinoleate HLB 4 (comme IMWITOR 600), polyglyceryl-3 polyricinoleate HLB: ~ 4 (comme Dermofeel^{®} PGPR de Evonik Dr. Straetmans GmbH) , polyglyceryl-2 sesquioleate HLB 4 (comme Dermofeel^{®} GO soft de Evonik Dr. Straetmans GmbH), polyglyceryl-2 diisostearate (comme Emulpharma^{®} PG20 de Res Pharma), polyglyceryl-3 diisostearate HLB: 5,5 (comme Plurol^{®} Diisostearique CG de Gattefossé, Lameform^{®} TGI de BASF, IMWITOR^{®} PG3 DIS de IOI Oleo GmbH, Cithrol^{™} PG32IS de Croda, DUB ISO G3 de Stéarinerie Dubois, Polyaldo^{®} 3-1-S de Lonza , Jolee 7245 d'Oléon et MASSOCARE PG3D de Masso) , polyglyceryl-3 oleate HLB 6.2 (comme I-MUL PGO 31 de Ivanhoe Industries), polyglyceryl-3 monostearate LB 7.2 , polyglyceryl-4 oleate HLB 8 (comme HYDRIOL^{®} PGMO.4 de HYDRIOR), polyglyceryl-5 dioleate HLB 8 (comme Dermofeel^{®} G 5 DO de Evonik Dr. Straetmans GmbH) , polyglyceryl-2 dipolyhydroxystearate (comme Dehymuls PGPH de Cognis) , polyglyceryl-2 diisostearate (comme Emulpharma PG20 de Respharma) , polyglyceryl-3 cocoate (comme Emulpharma^{®} Ecotech de Res Pharma).

Selon un mode préféré de réalisation, la composition selon l'invention comprend, à titre d'émulsionnant E/H, l'ester issu de la réaction de polyglycérol-3 et d'acide isostéarique (nom INCI : polyglyceryl-3 diisostearate).

L'émulsionnant E/H d'origine naturelle peut être constitués de mélanges d'esters gras, notamment d'esters gras de polyglycérols, d'esters gras de sorbitan ou d'esters de glucose comme en particulier des mélanges comme les produits Nikkomulse WO-NS de Nikko Chemicals (Polyglyceryl-6 polyricinoleate, polyglyceryl-2 isostearate, disteardimonium hectorite ), Tego Care LTP de Evonik (sorbitan laurate, polyglyceryl-4 laurate, dilauryl citrate), Sympatens-W/4500 (sorbitan oleate , polyglyceryl-3 polyricinoleate), Sympatens-O/2500 G (sorbitan stearate, methylglucose sesquistearate) , Symbio^{®}muls WO de Dr. Straetmans (polyglyceryl-3 polyricinoleate, sorbitan sesquioleate, cetyl ricinoleate, glyceryl caprate, cera alba, magnesium stearate, aluminum stearate), Ecomuls 2 in 1 de Natura-Tec (glyceryl oleate, polyglyceryl-3-polyricinoleate, olea europaea (olive) oil unsaponifiables) , HIPEgel Olea de Alchemy (glycerin, isopropyl palmitate, water, sucrose stearate, sucrose laurate).

Selon un mode préféré de réalisation, les émulsionnants mis en œuvre dans les compositions de l'invention sont d'origine naturelle. Le terme « d'origine naturelle » désigne toute molécule issue de ressources renouvelables, notamment extraite ou secrétée par des plantes, des micro-organismes ou d'algues et apte à permettre après modification physique, chimique ou enzymatique, susceptible d'être mise en œuvre dans une composition cosmétique.

Les tensioactifs mis en œuvre dans la composition selon l'invention sont présents en une teneur allant de 0,01 à 5% en poids, par rapport au poids total de la composition, de préférence de 0,1 à 1% en poids.

### Matière colorante

La composition selon l'invention peut encore comprendre au moins une matière colorante choisie parmi les colorants hydrosolubles ou liposolubles, les charges ayant pour effet de colorer et/ou opacifier la composition et/ou de colorer les lèvres, telles que les pigments, les nacres, les laques (colorants hydrosolubles adsorbés sur un support minéral inerte) et leurs mélanges. Ces matières colorantes peuvent être éventuellement traitées en surface par un agent hydrophobe tel que les silanes, silicones, savons d'acides gras, C9-15 fluoroalcool phosphates, copolymères acrylate/dimethicone, copolymères mixtes C9-15 fluoroalcool phosphates / silicones, lécithines, cire de carnauba, polyéthylène, chitosan et acides aminés éventuellement acylés tels que la lauroyl lysine, le disodium stearoyl glutamate et l'aluminium acyl glutamate. Les pigments peuvent être minéraux ou organiques, naturels ou de synthèse. Des exemples de pigments sont notamment les oxydes de fer, de titane ou de zinc, ainsi que les pigments composites et les pigments goniochromatiques, perlescents, interférentiels, photochromes ou thermochromes, sans que cette liste ne soit limitative.

Avantageusement, lorsqu'elle renferme un ou plusieurs pigments, la composition selon l'invention contient en outre au moins un dispersant tel que le cocoate de butylène glycol ou le malate de diisostéaryle.

### Charges

La composition utilisée selon l'invention peut en outre renfermer au moins une charge autre que la cyclodextrine précédemment décrite. Par ce terme, on entend toute particule de forme quelconque (notamment sphérique ou lamellaire), minérale ou organique, insoluble dans la composition. Des exemples de charges sont le talc, le mica, la silice, le kaolin, le nitrure de bore, l'amidon, l'amidon modifié par l'anhydride octénylsuccinique, les polyamides, les résines de silicone, les poudres d'élastomères de silicone et les poudres de polymères acryliques, en particulier de poly(méthacrylate de méthyle) ou les poudres de copolymère styrène acrylate (Sunsphere Powders de Dow). Les charges peuvent notamment être constituées de plusieurs couches de nature chimique et/ou de forme physique différentes et notamment se présenter sous forme de lamelles enrobées de charges sphériques. Elles peuvent être modifiées à l'aide de différents traitements de surface. Un exemple de charge traitée en surface est constitué par la silice modifiée par un copolymère éthylène / méthacrylate commercialisée notamment par la société KOBO sous les dénominations commerciales DSPCS 20N-I2, 3H-I2 et I2.

### Actifs

La composition utilisée selon l'invention peut par ailleurs contenir un ou plusieurs actifs hydrophiles ou lipophiles.

### Ingrédients cosmétiques usuels additionnels

La composition selon l'invention peut également comprendre tout ingrédient cosmétique usuel pouvant être choisi notamment parmi les antioxydants, les parfums, les conservateurs, les neutralisants, les tensioactifs, les filtres solaires, les vitamines, les hydratants, les composés auto-bronzants, les actifs antirides, les émollients, les actifs hydrophiles ou lipophiles, les agents anti-radicaux libres, les agents déodorants, les séquestrant, les agents filmogènes, et leurs mélanges.

En particulier, la composition selon l'invention peut comprendre au moins un filtre solaire.

### Les utilisations de cyclodextrine selon l'invention

Selon un premier mode de réalisation, l'un des objets de la présente invention porte aussi sur l'utilisation, dans une composition cosmétique ou dermatologique, d'au moins une cyclodextrine, au moins partiellement présente sous forme de particules solides dispersées dans un milieu physiologiquement acceptable, caractérisée en ce que les particules de cyclodextrine présentent une taille moyenne en volume d(4,3), mesurée par granulométrie laser, inférieure ou égale à 12 µm, et préférentiellement inférieure ou égale à 8, et une distribution de taille en volume, mesurée par granulométrie par diffraction laser, dont les diamètres caractéristiques d(10), d(50) et d(90) sont tels que :
a. le diamètre d(10) est inférieur ou égal à 2,5 µm,
b. le diamètre d(50) est inférieur ou égal à 10,0 µm, et
c. le diamètre d(90) est inférieur ou égal à 25,0 µm pour améliorer les propriétés sensorielles de ladite composition, préférentiellement pour conférer une facilité d'étalement, et/ou un toucher doux, poudré, non gras, et/ou prévenir la formation de peluches.

De plus, cette utilisation permet une nette diminution de la sensation de crissant à l'application, ainsi qu'une diminution, voire une disparation, du peluchage selon la nature des peaux.

En particulier, la cyclodextrine mise en œuvre dans cette utilisation est telle que décrite précédemment.

### Procédé de préparation d'une composition cosmétique selon l'invention

Un dernier objet de la présente invention consiste en un procédé de préparation d'une composition cosmétique, de préférence d'une émulsion huile-dans-eau, comprenant les étapes suivantes :
a) la fourniture d'un milieu physiologiquement acceptable,
b) La dispersion, dans ledit milieu physiologiquement acceptable, d'au moins une cyclodextrine présentant une taille moyenne en volume d(4,3), mesurée par granulométrie laser, inférieure ou égale à 12 µm, et préférentiellement inférieure ou égale à 8 et une distribution de taille en volume, mesurée par granulométrie par diffraction laser, dont les diamètres caractéristiques d(10), d(50) et d(90) sont tels que :
   a. le diamètre d(10) est inférieur ou égal à 2,5 µm,
   b. le diamètre d(50) est inférieur ou égal à 10,0 µm, et
   c. le diamètre d(90) est inférieur inférieur ou égal à 25,0 µm.

Selon une variante, le procédé selon l'invention est caractérisé en ce que la teneur en cyclodextrine dans le milieu physiologiquement acceptable est supérieure de 5% à sa solubilité dans ledit milieu, de préférence supérieure de 10% à sa solubilité, plus préférentiellement supérieure de 25% à sa solubilité, et plus préférentiellement supérieure de 50% à sa solubilité.

### Exemples

### Exemple 1 : émulsion

On a comparé une émulsion H/E réalisée selon l'invention avec une émulsion dite « de référence », réalisée en-dehors de l'invention, ayant la composition suivante :

### [Tableau 1]

**Tableau 1 : composition de l'émulsion**

| **Composant/ nom INCI** | **% massique** |
|---|---|
| Huile de tournesol / Helianthus annuus seed oil | 30 |
| Bêta-cyclodextrine / Cyclodextrin | 2,5 |
| Sorbitol (en poudre) | 1 |
| Polyglyceryl-3 diisostearate | 0,5 |
| Eau déminéralisée / Aqua | 64,3 |
| Gomme de xanthane / Xanthan gum | 0,7 |
| Conservateur | 1 |

L'émulsion de référence est obtenue en utilisant une bêta-cyclodextrine commercialisée sous le nom « Beauté by Roquette^{®} CD102 » par la demanderesse. L'émulsion selon l'invention est obtenue en utilisant cette même bêta-cyclodextrine préalablement broyée à sec pour présenter une taille moyenne en volume d(4,3) inférieure à 20 µm : on qualifie la bêta-cyclodextrine ainsi obtenue de « ultra-fine ». Les caractéristiques granulométriques de ces deux bêta-cyclodextrines sont présentées dans le tableau 2.

### [Tableau 2]

**Tableau 2 : caractéristiques granulométries des bêta-cyclodextrines**

| | **Beta-cyclodextrine Beauté by Roquette^{®} CD102 (référence)** | **Beta-cyclodextrine selon l'invention, dite « ultra-fine »** |
|---|---|---|
| d(4,3) (µm) | 94,83 | 11,43 |
| d10 (µm) | 21,81 | 2,378 |
| d50 (µm) | 85,81 | 9,716 |
| d90 (µm) | 182,2 | 22,85 |
| Coefficient de variation (%) | 64,7 % | 72,3 % |

Le protocole de préparation des émulsions est le suivant. On disperse un agent épaississant, en l'occurrence de la gomme de xanthane, dans de l'eau à 40°C sous agitation avec une pâles à défloculation à 500 tours par minute. A part, on mélange la bêta-cyclodextrine avec le sorbitol et le polyglycéryl-3 diisostearate. Ce mélange est alors ajouté dans l'eau contenant l'agent épaississant, sous agitation à 1000 tours par minute, afin d'obtenir une phase aqueuse.

La quantité de bêta-cyclodextrine est fixée à 2,5 % en masse de la composition pour les deux émulsions.

L'huile de tournesol constitue la phase huileuse, elle est chauffée à 40°C.

La phase huileuse est alors émulsionnée dans la phase aqueuse à 40°C sous agitation à 3000 tours par minute pendant 20 minutes.

On laisse refroidir et à température ambiante (20°C), on ajoute enfin un conservateur à base de phenoxyethanol.

Pour chacune des émulsions, on mesure des caractéristiques physico-chimiques, à savoir la viscosité et la taille des gouttelettes, et on mesure des caractéristiques sensorielles, à savoir l'opacité, la blancheur, la brillance, la fluidité, le filant, le glissant, le frais, le blanchissant, l'étalement, le gras, le collant, le doux, le crissant, le pénétrant, le poudré et le peluchage.

**La viscosité** est mesurée à l'aide d'un viscosimètre Brookfield DV-II+Pro. Un mobile de taille fixe (mobiles SP2 à SP7 utilisés selon les niveaux de viscosités conformément aux consignes de l'appareil) est mis en rotation à une vitesse de 20 tours par minute au contact de l'échantillon produit. La résistance du produit à ce mouvement de rotation est enregistrée pendant une minute et convertie en millipascal seconde. Pour chaque échantillon, la viscosité est mesurée trois fois et la moyenne arithmétique des trois valeurs est retenue.

La **taille des gouttelettes** est observée avec un microscope optique au grossissement x10. Le microscope utilisé est un LEICA DMLS.

Les **caractéristiques sensorielles** sont évaluées par un panel de dix personnes expertes dans l'analyse de la texture des produits cosmétiques. Les trois premiers descripteurs évalués sont des caractéristiques visuelles du produit. L'examen du produit se fait dans le pot, sous une lampe équipée d'une ampoule de température 5500 K.
- Le descripteur **fluide** est évalué lorsque qu'une pression est exercée sur le produit, entre le pouce et l'index. S'il n'a aucune tenue, qu'il s'écoule des doigts et s'écarte de la zone de pression, il est fluide, à l'inverse il est non fluide.
   Lors de l'étalement du produit, quatre autres descripteurs sont évalués. L'examen du produit se fait sous la lampe, après avoir déposé sur la main 50 à 100 µL du produit étudié, pendant son étalement en 10 rotations.
- Le descripteur **poudré** est évalué entre la 2^{ème} et la 5^{ème} rotation. Les doigts glissent bien sur la peau. Le produit est perçu comme une substance poudreuse sur la peau.
- **L'étalement** est évalué en examinant le produit après avoir déposé sur la main 50 à 100 µL du produit, pendant son étalement en 10 rotations, sous une lampe. L'étalement est d'autant plus important qu'il y a peu de résistance au mouvement entre le 5 ème et le 10 ème tour sur la main.

Les derniers descripteurs sont évalués après avoir effectué les 10 rotations.
Pour les quatre descripteurs suivants, l'examen se fait sous la lampe, sur la peau, 1 minute après l'étalement de 50 à 100µl du produit.
- Le descripteur doux est évalué en effectuant un glissement sur la peau, un toucher sec et glissant est ressenti.
- Le descripteur crissant est évalué en effectuant un glissement du pouce sur l'index, un frein est ressenti et un bruit crissant est entendu.
   Pour les deux descripteurs suivants, l'examen se fait sous la lampe, sur la peau, 2 minutes après l'étalement de 50 à 100 µL du produit.
- Le descripteur pénétrant du produit est évalué en effectuant un glissement sur la peau. Un panel d'évaluateurs évalue alors la quantité de résidu de produit récupéré.
- Le peluchage est évalué en effectuant une action mécanique de frottement sur la peau, le produit entraine la formation de peluches.

### [Tableau 3]

**Tableau 3 : Perceptions sensorielles améliorées par rapport à l'émulsion de référence**

| **Perception sensorielle** | **Emulsion selon l'invention, avec beta-cyclodextrine ultra-fine, par rapport à l'émulsion avec Beauté by Roquette^{®} CD102** |
|---|---|
| Fluide | Moins fluide |
| Poudré | Plus poudré |
| Etalement | Plus facile |
| Doux | Toucher plus doux |
| Crissant | Nettement moins crissant |
| Pénétrant | Pénétration plus rapide |
| Peluchage | Peluches réduites ou absentes |

Les critères de propriétés sensorielles améliorés dans l'émulsion selon l'invention sont : fluide, poudré, étalement, gras, doux, crissant, pénétrant, peluchage.

### Exemple 2 : crème solaire

On a comparé le profil sensoriel d'une formulation de crème solaire selon qu'on la prépare avec une bêta-cyclodextrine de diamètre moyen 200 µm (Beauté by Roquette^{®} CD102 de l'exemple 1) ou avec une bêta-cyclodextrine de taille moyenne en volume de 11,43 µm (bêta-cyclodextrine ultra-fine de l'exemple 1), selon le mode opératoire ci-après.

On commence par préparer la phase A1 selon la composition du tableau 4 : on disperse la poudre Sunsphere dans l'eau et le Cetiol C5, puis on chauffe à 55°C sous agitation à 2000 rpm avec un rotor-stator pendant 15 minutes.

### [Tableau 4]

**Tableau 4 : composition de la phase A1**

| Phase | Nom commercial | Fournisseur | Nom INCI | %m au total |
|---|---|---|---|---|
| A1 | Eau déminéralisée | Cooper | Aqua | 36,44 |
| | EDETA BD | AMI Chimie | Disodium EDTA | 0,10 |
| | Sunsphere powder | Dow (Univar) | Styrene/acrylate copolymer | 5,00 |
| | Cetiol C5 | AMI Chimie | Coco-caprylate | 1,00 |

A part, on prépare la phase A2, en pesant tous les ingrédients dans une coupelle, puis on l'ajoute dans la phase A1 sous agitation rotor-stator à 2000 rpm pendant 15 min. On conserve la phase A1+A2 à 70°C sous agitation.

### [Tableau 5]

**Tableau 5 : composition de la phase A2**

| Phase | Nom commercial | Fournisseur | Nom INCI | %m au total |
|---|---|---|---|---|
| A2 | Sepinov WEO | Seppic | Hydroxymethyl Acrylate/Sodium Acryloyldimethyl Taurate Copolymer | 0,20 |
| | Microcare PHC | Thor | Glycerin & Chlorphenesin & Phenoxyethanol | 1,00 |
| | Gomme de xanthane | Interchimie | Xanthan gum | 0,30 |
| | Glycérine codex | Cooper | Glycerin | 3,00 |
| | Romol AFSK | Saci cfpa | Potassium cetyl phosphate | 0,30 |

On prépare ensuite la phase B1 en chauffant à 70°C sous agitation magnétique pendant 10 minutes selon la composition du tableau 6.

### [Tableau 6]

**Tableau 6 : composition de la phase B1**

| Phase | Nom commercial | Fournisseur | Nom INCI | %m au total |
|---|---|---|---|---|
| B1 | Cetiol C5 | AMI Chimie | Coco-caprylate | 2,00 |
| | Cocoate BG | Gattefossé | Butylene glycol cocoate | 2,00 |
| | Parsol 1789 | IES Ingrédients | Butyl MethoxyDibenzoylMethan | 5,00 |
| | Parsol EHS | IES Ingrédients | Ethylhexyl Salicylate | 5,00 |
| | Parsol MCX | IES Ingrédients | Ethylhexyl Methoxycinnamate | 7,50 |
| | Parsol HMS | IES Ingrédients | Homosalate | 9,00 |
| | Solastay S1 | Azelis | Ethylhexyl methoxycrylene | 3,00 |
| | Hallbrite BHB | Azelis | Butyl Octyl Salycilate | 4,00 |
| | Tinogard TL | AMI Chimie | Benzotriazolyl Dodecyl P-Cresol | 1,00 |
| | Covanol Red ON 3780 | Sensient | Iron oxides (CI77491) & Octyldodecanol & Stearic acid & Magnesium hydroxide & Aluminium hydroxide & Sorbitan oleate | 0,12 |
| | Covanol Yellow ON 1782 | Sensient | Iron oxides (CI77492) & Octyldodecanol & Stearic acid & Magnesium hydroxide & Aluminium hydroxide & Sorbitan oleate | 0,53 |
| | Huile de tournesol oléique | Oléon | Helianthus annuus seed oil | 3,00 |

En conservant la phase B1 à 70°C sous agitation, on ajoute la phase B2, préparée à part et constituée selon la composition du tableau 7, et on maintient sous agitation pendant 10 minutes, en suite de quoi, on procède à l'émulsification de la phase B1+B2 dans la phase A.

### [Tableau 7]

**Tableau 7 : composition de la phase B2**

| Phase | Nom commercial | Fournisseur | Nom INCI | %m au total |
|---|---|---|---|---|
| B2 | Beauté by Roquette^{®} CD102 | Roquette Frères | Cyclodextrin | 2,5 |
| | Plurol | Gattefossé | Polyglyceryl 3-diisostearate | 0,5 |
| | Neosorb | Roquette Frères | Sorbitol | 1 |

Pour émulsionner la phase B1+B2 dans la phase A, on verse la totalité de la phase B1+B2 dans la phase A à 55°C sous agitation au rotor-stator à 2 500 rpm, puis on maintient pendant 5 minutes à 55°C, et pendant 10 minutes sous bain d'eau froide. On réduit la vitesse à 1200 rpm et on continue d'agiter jusqu'à ce que l'émulsion soit à température ambiante sous bain d'eau froide.

On ajoute ensuite la phase C, puis on colore avec la phase D.

On obtient alors une crème teintée et parfumée. Lors de son application sur la peau, la crème peluche, elle est confortable mais un effet crissant est noté lors de son étalement.

### [Tableau 8]

**Tableau 8 : composition des phases C et D**

| Phase | Nom commercial | Fournisseur | Nom INCI | %m au total |
|---|---|---|---|---|
| C | Plouf RL G 113 23705 | Robertet | Parfum | 0,50 |
| D | Covarine white WN 9787 | Sensient | CI 77891 (and) Glycerin (and) Xanthan Gum (and) Sodium Citrate (and) Aqua | 4,00 |
| | Covarine black WN 9798 | Sensient | CI 77499 & Glycerin & Xanthan gum & Sodium citrate & Aqua | 0,01 |

On prépare une crème solaire selon le protocole précédent en substituant la « Beauté by Roquette^{®} CD102 » par la bêta-cyclodextrine « ultra-fine » de l'exemple 1. On applique la crème sur la peau : elle ne peluche plus, la crème est plus confortable. Un léger effet crissant persiste à la fin de la pénétration de la crème, mais il est nettement moins présent qu'avec la formulation de référence comprenant la bêta-cyclodextrine « Beauté by Roquette^{®} CD102 ».

## Revendications

1. Composition cosmétique ou dermatologique comprenant au moins une cyclodextrine présente, au moins partiellement sous forme de particules solides dispersées dans un milieu physiologiquement acceptable, **caractérisée en ce que** les particules de cyclodextrine présentent :
- une taille moyenne en volume d(4,3), mesurée par granulométrie laser, inférieure ou égale à 12 µm, préférentiellement inférieure ou égale à 8 µm, et
- une distribution de taille en volume, mesurée par granulométrie par diffraction laser, dont les diamètres caractéristiques d(10), d(50) et d(90) sont tels que :
a. Le diamètre d(10) est inférieur ou égal à 2,5 µm,
b. Et le diamètre d(50) est inférieur ou égal à 10,0 µm,
c. Et le diamètre d(90) est inférieur ou égal à 25,0 µm,
**caractérisée en ce que** les particules solides de cyclodextrine présentent une distribution de taille en volume, mesurée par granulométrie par diffraction laser, ayant un coefficient de variation inférieur ou égal à 73 %.

2. Composition cosmétique ou dermatologique selon la revendication 1, **caractérisée en ce qu'**au moins 5 % en poids de cyclodextrine, par rapport au poids total de cyclodextrine présente dans la composition, est sous forme de particules solides, préférentiellement au moins 10 % en poids, plus préférentiellement au moins 15 % en poids, plus préférentiellement au moins 25 % en poids, et encore plus préférentiellement au moins 50% en poids.

3. Composition cosmétique ou dermatologique selon l'une des revendications précédentes, **caractérisée en ce que** la teneur en cyclodextrine dans la composition est supérieure de 5% à sa solubilité dans le milieu physiologiquement acceptable de ladite composition, de préférence supérieure de 10% à sa solubilité dans ledit milieu, plus préférentiellement supérieure de 15 % à sa solubilité dans ledit milieu, tout préférentiellement supérieure de 25% à sa solubilité dans ledit milieu, et plus préférentiellement supérieure de 50% à sa solubilité dans ledit milieu.

4. Composition cosmétique ou dermatologique selon l'une des revendications précédentes **caractérisée en ce que** la cyclodextrine est une bêta-cyclodextrine, de préférence native .

5. Composition cosmétique ou dermatologique selon l'une des revendications précédentes, **caractérisée en ce qu'**elle constitue un produit de soin de la peau, un soin des cheveux, un soin bucco-dentaire, un maquillage, ou un soin dépilatoire.

6. Utilisation, d'au moins une cyclodextrine, au moins partiellement présente sous forme de particules solides dispersées dans un milieu physiologiquement acceptable, dans une composition cosmétique ou dermatologique, **caractérisée en ce que** les particules de cyclodextrine présentent :
- une taille moyenne en volume d(4,3), mesurée par granulométrie laser, inférieure ou égale à 12 µm, préférentiellement inférieure ou égale à 8, et
- une distribution de taille en volume, mesurée par granulométrie par diffraction laser, dont les diamètres caractéristiques d(10), d(50) et d(90) sont tels que :
a. Le diamètre d(10) est inférieur ou égal à 2,5 µm,
b. Et le diamètre d(50) est inférieur ou égal à 10,0 µm,
c. Et le diamètre d(90) est inférieur ou égal à25,0 µm,
**caractérisée en ce que** les particules solides de cyclodextrine présentent une distribution de taille en volume, mesurée par granulométrie par diffraction laser, ayant un coefficient de variation inférieur ou égal à 73 %,
pour améliorer les propriétés sensorielles de ladite composition, préférentiellement pour conférer une facilité d'étalement, et/ou un toucher doux, poudré, non gras, et/ou prévenir la formation de peluches.

7. Utilisation selon la revendication 6, **caractérisée en ce que** la cyclodextrine est une bêta-cyclodextrine native.

8. Utilisation selon l'une des revendications 6 ou 7, **caractérisée en ce que** les compositions cosmétiques ou dermatologiques sont choisies parmi un produit de soin de la peau, un soin des cheveux, un soin bucco-dentaire, un maquillage, ou un soin dépilatoire.

9. Utilisation selon la revendication 8, **caractérisée en ce que** les compositions cosmétiques se présentent sous la forme de gels, d'émulsions, de suspensions, de lotions, de sérums, d'aérosols, de mousses, de pâtes, de poudres, ou de sticks, et préférentiellement d'émulsions.

10. Procédé de préparation d'une composition cosmétique ou dermatologique, de préférence d'une émulsion huile-dans-eau, comprenant les étapes suivantes :
a. la fourniture d'un milieu physiologiquement acceptable,
b. La dispersion, dans ledit milieu physiologiquement acceptable, d'au moins une cyclodextrine présentant une taille moyenne en volume d(4,3), mesurée par granulométrie laser, inférieure ou égale à 12 µm, préférentiellement inférieure ou égale à 8 et une distribution de taille en volume, mesurée par granulométrie par diffraction laser, dont les diamètres caractéristiques d(10), d(50) et d(90) sont tels que :
a. Le diamètre d(10) est inférieur ou égal à 2,5 µm,
b. Et le diamètre d(50) est inférieur ou égal à 10,0 µm,
c. Et le diamètre d(90) est inférieur ou égal à 25,0 µm,
**caractérisée en ce que** les particules solides de cyclodextrine présentent une distribution de taille en volume, mesurée par granulométrie par diffraction laser, ayant un coefficient de variation inférieur ou égal à 73 %.

11. Procédé selon la revendication précédente, **caractérisé en ce que** la teneur en cyclodextrine dans le milieu est supérieure de 5% à sa solubilité dans ledit milieu physiologiquement acceptable, de préférence supérieure de 10% à sa solubilité, plus préférentiellement supérieure de 25% à sa solubilité, et plus préférentiellement supérieure de 50% à sa solubilité.

## Patentansprüche

1. Kosmetische oder dermatologische Zusammensetzung, die wenigstens ein Cyclodextrin enthält, das wenigstens teilweise in Form von festen Partikeln vorliegt, die in einem physiologisch akzeptablen Medium dispergiert sind, **dadurch gekennzeichnet, dass** die Cyclodextrinpartikel aufweisen:
- eine mittlere Volumengröße d(4,3), gemessen durch Lasergranulometrie, von 12 µm oder weniger, bevorzugt von 8 µm oder weniger, und
- eine Volumengrößenverteilung, gemessen durch Laserbeugungsgranulometrie, mit charakteristischen Durchmessern d(10), d(50) und d(90), die wie folgt sind:
a. Der Durchmesser d(10) ist kleiner oder gleich 2,5 µm,
b. und der Durchmesser d(50) ist kleiner oder gleich 10,0 µm,
c. und der Durchmesser d(90) ist kleiner oder gleich 25,0 µm,
**dadurch gekennzeichnet, dass** die festen Cyclodextrinpartikel eine Volumengrößenverteilung, gemessen durch Laserbeugungsgranulometrie, mit einem Variationskoeffizienten von kleiner oder gleich 73 % aufweisen.

2. Kosmetische oder dermatologische Zusammensetzung nach Anspruch 1, **dadurch gekennzeichnet, dass** wenigstens 5 Gew.-% Cyclodextrin, bezogen auf das Gesamtgewicht des in der Zusammensetzung vorhandenen Cyclodextrins, in Form von festen Partikeln vorliegen, bevorzugt wenigstens 10 Gew.-%, stärker bevorzugt wenigstens 15 Gew.-%, stärker bevorzugt wenigstens 25 Gew.-% und noch stärker bevorzugt wenigstens 50 Gew.-%.

3. Kosmetische oder dermatologische Zusammensetzung nach einem der vorhergehenden Ansprüche, **dadurch gekennzeichnet, dass** der Gehalt an Cyclodextrin in der Zusammensetzung 5 % über seiner Löslichkeit in dem physiologisch akzeptablen Medium der Zusammensetzung liegt, bevorzugt 10 % über seiner Löslichkeit in diesem Medium, stärker bevorzugt 15 % über seiner Löslichkeit in diesem Medium, ganz besonders bevorzugt 25 % über seiner Löslichkeit in diesem Medium und noch stärker bevorzugt 50 % über seiner Löslichkeit in diesem Medium liegt.

4. Kosmetische oder dermatologische Zusammensetzung nach einem der vorhergehenden Ansprüche, **dadurch gekennzeichnet, dass** das Cyclodextrin ein Beta-Cyclodextrin ist, bevorzugt nativ.

5. Kosmetische oder dermatologische Zusammensetzung nach einem der vorhergehenden Ansprüche, **dadurch gekennzeichnet, dass** sie ein Hautpflegemittel, ein Haarpflegemittel, ein Mundpflegemittel, ein Make-up oder ein Enthaarungsmittel ist.

6. Verwendung wenigstens eines Cyclodextrins, das wenigstens teilweise in Form von festen Partikeln vorliegt, die in einem physiologisch akzeptablen Medium dispergiert sind, in einer kosmetischen oder dermatologischen Zusammensetzung, **dadurch gekennzeichnet, dass** die Cyclodextrinpartikel aufweisen:
- eine mittlere Volumengröße d(4,3), gemessen durch Lasergranulometrie, von 12 µm oder weniger, bevorzugt von 8 µm oder weniger, und
- eine Volumengrößenverteilung, gemessen durch Laserbeugungsgranulometrie, mit charakteristischen Durchmessern d(10), d(50) und d(90), die wie folgt sind:
a. Der Durchmesser d(10) ist kleiner oder gleich 2,5 µm,
b. und der Durchmesser d(50) ist kleiner oder gleich 10,0 µm,
c. und der Durchmesser d(90) ist kleiner oder gleich 25,0 µm,
**dadurch gekennzeichnet, dass** die festen Cyclodextrinpartikel eine Volumengrößenverteilung, gemessen durch Laserbeugungsgranulometrie, mit einem Variationskoeffizienten von 73 % oder weniger aufweisen, um die sensorischen Eigenschaften der Zusammensetzung zu verbessern, bevorzugt um eine leichte Verteilbarkeit und/oder einen weichen, pudrigen, nicht fettenden Griff zu verleihen und/oder die Bildung von Fusseln zu verhindern.

7. Verwendung nach Anspruch 6, **dadurch gekennzeichnet, dass** das Cyclodextrin ein natives Beta-Cyclodextrin ist.

8. Verwendung nach einem der Ansprüche 6 oder 7, **dadurch gekennzeichnet, dass** die kosmetischen oder dermatologischen Zusammensetzungen gewählt sind aus einem Hautpflegemittel, einem Haarpflegemittel, einem Mundpflegemittel, einem Make-up oder einem Enthaarungsmittel.

9. Verwendung nach Anspruch 8, **dadurch gekennzeichnet, dass** die kosmetischen Zusammensetzungen in Form von Gelen, Emulsionen, Suspensionen, Lotionen, Seren, Aerosolen, Schäumen, Pasten, Pulvern oder Sticks und bevorzugt in Form von Emulsionen vorliegen.

10. Verfahren zur Herstellung einer kosmetischen oder dermatologischen Zusammensetzung, bevorzugt einer Öl-in-Wasser-Emulsion, umfassend die folgenden Schritte:
a. Bereitstellung eines physiologisch akzeptablen Mediums,
b. Dispergieren wenigstens eines Cyclodextrins in dem physiologisch akzeptablen Medium, das eine mittlere Volumengröße d(4,3), gemessen durch Lasergranulometrie, von kleiner gleich 12 µm, bevorzugt kleiner gleich 8, und eine Volumengrößenverteilung, gemessen durch Laserbeugungsgranulometrie, aufweist, deren charakteristische Durchmesser d(10), d(50) und d(90) wie folgt sind:
a. Der Durchmesser d(10) ist kleiner oder gleich 2,5 µm,
b. und der Durchmesser d(50) ist kleiner oder gleich 10,0 µm,
c. und der Durchmesser d(90) ist kleiner oder gleich 25,0 µm,
**dadurch gekennzeichnet, dass** die festen Cyclodextrinpartikel eine Volumengrößenverteilung, gemessen durch Laserbeugungsgranulometrie, mit einem Variationskoeffizienten von 73% oder weniger aufweisen.

11. Verfahren nach dem vorhergehenden Anspruch, **dadurch gekennzeichnet, dass** der Gehalt an Cyclodextrin im Medium 5 % über seiner Löslichkeit in dem physiologisch akzeptablen Medium liegt, bevorzugt 10 % über seiner Löslichkeit, stärker bevorzugt 25 % über seiner Löslichkeit und noch stärker bevorzugt 50 % über seiner Löslichkeit liegt.

## Claims

1. Cosmetic or dermatological composition comprising at least one cyclodextrin, which is at least partially present in the form of solid particles dispersed in a physiologically acceptable medium, **characterised in that** the cyclodextrin particles have:
- a volume average size d(4.3), measured by laser granulometry, of less than or equal to 12 µm, preferably less than or equal to 8 µm, and
- a size distribution by volume, measured by laser diffraction granulometry, of which the characteristic diameters d(10), d(50) and d(90) are such that:
a. the diameter d(10) is less than or equal to 2.5 µm,
b. and the diameter d(50) is less than or equal to 10.0 µm,
c. and the diameter d(90) is less than or equal to 25.0 µm,
**characterised in that** the solid cyclodextrin particles have a size distribution by volume, measured by laser diffraction granulometry, having a coefficient of variation of less than or equal to 73%.

2. Cosmetic or dermatological composition according to claim 1, **characterised in that** at least 5% by weight of cyclodextrin, relative to the total weight of cyclodextrin present in the composition, is in the form of solid particles, preferably at least 10% by weight, more preferably at least 15% by weight, more preferably at least 25% by weight, and even more preferably at least 50% by weight.

3. Cosmetic or dermatological composition according to any one of the preceding claims, **characterised in that** the cyclodextrin content in the composition is 5% greater than its solubility in the physiologically acceptable medium of said composition, preferably 10% greater than its solubility in said medium, more preferably 15% greater than its solubility in said medium, very preferably 25% greater than its solubility in said medium, and more preferably 50% greater than its solubility in said medium.

4. Cosmetic or dermatological composition according to one of the preceding claims, **characterised in that** the cyclodextrin is a beta-cyclodextrin, preferably native.

5. Cosmetic or dermatological composition according to any one of the preceding claims, **characterised in that** it forms a skincare product, a haircare product, an oral-dental care product, a make-up product, or a hair removal product.

6. Use of at least one cyclodextrin, at least partially present in the form of solid particles dispersed in a physiologically acceptable medium, in a cosmetic or dermatological composition, **characterised in that** the cyclodextrin particles have:
- a volume average size d(4.3), measured by laser granulometry, of less than or equal to 12 µm, preferably less than or equal to 8, and
- a size distribution by volume, measured by laser diffraction granulometry, of which the characteristic diameters d(10), d(50) and d(90) are such that:
a. the diameter d(10) is less than or equal to 2.5 µm,
b. and the diameter d(50) is less than or equal to 10.0 µm,
c. and the diameter d(90) is less than or equal to 25.0 µm,
**characterised in that** the solid cyclodextrin particles have a size distribution by volume, measured by laser diffraction granulometry, having a coefficient of variation of less than or equal to 73%,
to improve the sensory properties of said composition, preferably to impart ease of spreading, and/or a soft, powdery, non-greasy feel, and/or prevent pilling.

7. Use according to claim 6, **characterised in that** the cyclodextrin is a native beta-cyclodextrin.

8. Use according to one of claims 6 or 7, **characterised in that** the cosmetic or dermatological compositions are selected from a skincare product, a haircare product, an oral-dental care product, a make-up product, or a hair removal product.

9. Use according to claim 8, **characterised in that** the cosmetic compositions are in the form of gels, emulsions, suspensions, lotions, serums, aerosols, foams, pastes, powders or sticks, and preferably emulsions.

10. Method for preparing a cosmetic or dermatological composition, preferably an oil-in-water emulsion, comprising the following steps:
a. providing a physiologically acceptable medium,
b. dispersing, in said physiologically acceptable medium, at least one cyclodextrin having a volume average size d(4.3), measured by laser granulometry, of less than or equal to 12 µm, preferably less than or equal to 8 and a volume size distribution, measured by laser diffraction particle size, of which the characteristic diameters d(10), d(50) and d(90) are such that:
a. the diameter d(10) is less than or equal to 2.5 µm,
b. and the diameter d(50) is less than or equal to 10.0 µm,
c. and the diameter d(90) is less than or equal to 25.0 µm,
**characterised in that** the solid cyclodextrin particles have a size distribution by volume, measured by laser diffraction granulometry, having a coefficient of variation of less than or equal to 73%.

11. Method according to the preceding claim, **characterised in that** the cyclodextrin content in the medium is 5% greater than its solubility in said physiologically acceptable medium, preferably 10% greater than its solubility, more preferably 25% greater than its solubility, and more preferably 50% greater than its solubility.
